# FASCICULE DE BREVET EUROPEEN

(11) **EP 2 249 724 B1**
(45) Date de publication et mention de la délivrance du brevet: **11.12.2013**
(21) Numéro de dépôt: 09722874.6
(22) Date de dépôt: 04.02.2009
(51) Int. Cl.: A61B 17/70

(54) **Élément de stabilisation dynamique de vertèbres**
Element zur dynamischen Stabilisierung von Wirbeln
Member for dynamic stabilisation of vertebrae

(30) Priorité: 04.02.2008 FR 0850669
(43) Date de publication de la demande: 17.11.2010
(73) Titulaire: Spinevision, 75012 Paris (FR)
(72) Inventeur: DROULOUT, Thomas, F-78 300 POISSY (FR); PETIT, Dominique, F-62180 Verton (FR)
(74) Mandataire: Breesé, Pierre
(86) Numéro de dépôt international: PCT/FR2009/000126
(87) Numéro de publication internationale: WO 2009/115663

(56) Documents cités:
- WO-A-2005/092222
- WO-A-2006/079531
- DE-A1-102004 048 938
- US-A- 4 697 582
- US-A1- 2004 143 264
- US-A1- 2007 233 064

## Description

L'invention concerne le domaine de la stabilisation dynamique des vertèbres.

L'invention concerne plus particulièrement un élément de stabilisation dynamique de vertèbres avoisinantes, destiné à coopérer avec au moins deux ensembles de connexion rachidienne implantables sur une vertèbre.

De manière générale, les éléments de stabilisation dynamique sont destinés à réaligner les vertèbres les unes par rapport aux autres tout en réduisant les contraintes sur les facettes articulaires et sur les disques intervertébraux en autorisant certains mouvements des vertèbres.

On connaît de l'art antérieur de tels éléments de stabilisation dynamique.

En particulier, on connaît de la demande de brevet internationale WO2004/024011 un élément de liaison dynamique constitué, au moins partiellement, d'un support en matériau polymère et de deux tiges : une première tige sensiblement coaxiale avec le support et une seconde tige formée de spires entourant la première tige, lesdites spires étant au moins partiellement noyées dans le support.

On connaît également de la demande de brevet internationale WO2005/087121 un élément de liaison souple comportant un câble entouré au moins en partie par une enveloppe en polymère, ledit câble étant constitué d'au moins un brin élastique coaxial avec ladite enveloppe.

Le document US 2004/0143264 A1 divulgue un élément de stabilisation dynamique ayant les caractéristiques définies dans la préambule de la revendication 1.

Destinés à restaurer l'alignement des vertèbres, les éléments de stabilisation sont fixés à celles-ci par l'intermédiaire d'ensembles de connexions implantables. De manière conventionnelle, les ensembles de connexion comprennent un moyen d'ancrage osseux agencé pour recevoir l'élément de stabilisation dynamique. La fixation de l'élément de stabilisation dynamique sur le moyen d'ancrage est réalisée au moyen d'une pièce complémentaire de fermeture. Ainsi, l'élément de stabilisation dynamique est maintenu entre le moyen d'ancrage osseux et la pièce de fermeture. L'élément de stabilisation dynamique est maintenu fixe sur le moyen d'ancrage par serrage de l'élément de stabilisation dynamique contre le moyen d'ancrage osseux. Le serrage est réalisé généralement au moyen d'un écrou lequel est disposé en contact avec l'élément de stabilisation dynamique. Sous l'action du serrage de l'écrou, l'élément de stabilisation dynamique est pressé contre le moyen d'ancrage.

Afin d'effectuer le serrage de l'écrou sur l'élément de stabilisation dynamique et ainsi permettre le maintien de ce dernier sur le moyen d'ancrage, il est usuel de prévoir, entre l'écrou et l'élément de stabilisation dynamique, une bague de protection rigide. La présence d'une bague de protection entre l'écrou et l'élément de liaison dynamique évite en effet que celui-ci ne subisse une déformation plastique du fait de l'opération de serrage.

Cette configuration nécessite cependant d'adapter la longueur de l'élément de stabilisation, ainsi que de prévoir une position précise des bagues de protection sur l'élément de stabilisation dynamique suivant la position des moyens des moyens d'ancrage. Cela conduit alors à des mises en place des éléments de stabilisation dynamique pouvant s'avérer longues et fastidieuses.

L'invention vise notamment à pallier l'inconvénient précédemment décrit en proposant un élément de stabilisation pouvant être rapidement positionné sur les moyens d'ancrage tout en garantissant le comportement élastique, ou du moins le comportement flexible, souhaité entre les moyens d'ancrage.

A cet effet, et selon un premier aspect, l'invention concerne un élément de stabilisation dynamique pour vertèbres selon la revendication 1.

La présence d'une gaine de fixation rigide permet d'assurer et de maintenir le serrage de l'élément de stabilisation dynamique sur les moyens d'ancrage tout en autorisant les mouvements d'extension, de compression ainsi que de flexion par la présence d'espaces entre les zones rigides de la gaine de fixation.

La gaine de fixation ainsi formée protège la partie souple de l'élément de stabilisation en tout point de sa longueur, tout en conservant les propriétés de flexion, de distraction et/ou de compression dudit élément conférées par la constitution même de la tige.

Avantageusement, les zones rigides sont espacées les unes des autres d'une distance inférieure à la longueur nominale d'une zone de contact définie par le moyen de serrage avec l'élément de stabilisation dynamique.

L'élément pourvu d'une telle gaine présente en outre l'avantage de pouvoir être rapidement mis en place sur les moyens d'ancrage fixés sur les vertèbres. En effet, la distance imposée entre les zones rigides fait que le moyen de serrage est pour l'essentiel en contact avec les zones rigides. L'élément de stabilisation dynamique ne requiert donc aucun positionnement précis sur les moyens d'ancrage.

Selon une configuration particulière, les zones rigides consistent en des anneaux distincts espacés les uns des autres d'une distance inférieure à la longueur de la zone de contact.

Selon une autre configuration, la gaine de fixation consiste en une bande hélicoïdale comprenant des spires s'étendant autour de la tige selon un axe sensiblement coaxial avec l'axe longitudinal de la tige de liaison, lesdites spires formant les zones rigides de la gaine de fixation.

Avantageusement, l'élément de stabilisation dynamique comprend des moyens de maintien de la gaine de fixation sur la tige positionnés entre les zones rigides de la gaine de fixation.

Il est également prévu, afin d'amortir un mouvement en compression de l'élément de stabilisation dynamique, des anneaux d'amortissement en compression, chaque anneau étant intercalé entre deux zones rigides adjacentes de la gaine de fixation. Fixation selon une configuration particulière, les anneaux d'amortissement forment les moyens de maintien de la gaine de fixation. Par ailleurs, les anneaux d'amortissement sont constitués par des renflements radiaux de l'enveloppe.

Ainsi, lors de la réalisation de l'élément de stabilisation dynamique par moulage du matériau élastique autour du câble, le matériau élastique destiné à former l'enveloppe se répartit dans les ouvertures formées dans la gaine, à savoir les espacements formés entre les zones rigides. « Emprisonnée » dans la matière constituant l'enveloppe, la gaine de fixation est ainsi fermement maintenue. Le glissement de la gaine de fixation est donc empêché du fait de l'extension de la matière plastique.

Il peut être également prévu que les zones rigides comprennent une ou plusieurs lumières. La présence de lumières vient renforcer la fonction de maintien des parties « débordantes » de l'enveloppe (extensions). Une telle configuration est particulièrement avantageuse notamment lorsque la gaine de fixation est formée par des anneaux distincts.

Selon une configuration particulière de l'élément de stabilisation dynamique, les zones rigides sont équidistantes les unes des autres.

Afin d'améliorer sa résistance globale, les extrémités libres de l'élément de stabilisation dynamique sont pourvues d'un embout rigide. Selon un mode de réalisation particulier, les embouts sont fixés, de préférence par soudage ou emboutissage, aux extrémités du câble.

Selon un deuxième aspect, l'invention concerne un élément de liaison comprenant au moins un élément de stabilisation dynamique tel que décrit précédemment, l'élément de liaison étant prolongé par au moins une tige rigide. Selon l'application souhaitée, il pourra être avantageux de prévoir dans le prolongement de l'une ou les deux extrémités de la tige de l'élément de stabilisation dynamique prolongée une tige rigide. Il sera ainsi possible d'assurer, avec un seul et même élément de liaison, à la fois une liaison d'ostéosynthèse et une liaison dynamique.

Selon un autre aspect, l'invention concerne un système de fixation rachidien comportant au moins deux ensembles de connexion rachidienne implantables, deux ensembles au moins étant reliés par un élément de stabilisation dynamique tel que décrit précédemment.

D'autres objets et avantages de l'invention apparaîtront au cours de la description qui suit, faite en référence aux dessins annexés, dans lesquels :
- la figure 1 illustre une vue partielle en perspective d'un système de fixation rachidien comprenant un élément de stabilisation dynamique selon une première configuration de l'invention maintenu par deux ensembles de connexion implantables ;
- la figure 2 est une vue schématique partielle de côté d'un l'élément de stabilisation dynamique selon l'invention lequel est au contact de moyens de serrage d'ensembles de connexion ;
- la figure 3 illustre une vue en coupe transversale partielle de l'élément de stabilisation dynamique de la figure 2 selon l'axe III-III ;
- la figure 4 illustre un élément de stabilisation dynamique selon une deuxième configuration de l'invention ;
- les figures 5a, 5b, 5c illustrent un élément de liaison hybride comprenant au moins une partie de stabilisation dynamique ;
- les figures 6a et 6b illustrent respectivement une vue schématique en perspective d'un élément de stabilisation dynamique selon une troisième configuration de l'invention, avec et sans son enveloppe ;
- la figure 7 illustre une vue d'un élément de stabilisation dynamique selon une quatrième configuration de l'invention, lequel est sans son enveloppe ;
- les figures 8a et 8b illustrent respectivement une vue schématique en perspective d'un élément de stabilisation dynamique selon une cinquième configuration de l'invention, avec et sans son enveloppe ; et
- la figure 9 illustre une vue d'un élément de stabilisation dynamique selon une sixième configuration de l'invention, lequel est représenté sans son enveloppe.

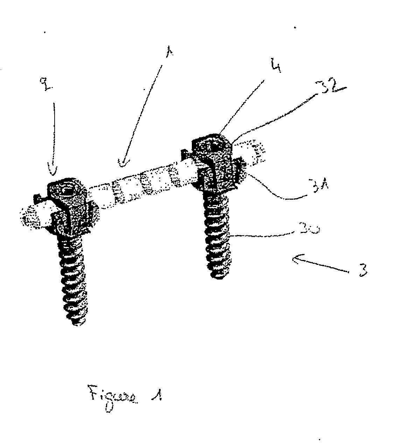

En relation avec les figures 1 à 3, il est décrit un élément de stabilisation dynamique 1 de vertèbres avoisinantes. L'élément de stabilisation dynamique 1 est destiné à être maintenu le long des vertèbres au moyen d'au moins deux ensembles de connexion rachidienne implantables 2.

De manière classique en soi, un ensemble de connexion 2 comprend un moyen d'ancrage osseux 3 agencé pour recevoir l'élément de stabilisation dynamique 1 et un moyen de serrage 4 de l'élément de stabilisation dynamique 1 sur ledit moyen d'ancrage 2.

Dans le mode de réalisation décrit, le moyen d'ancrage 3 comprend une partie filetée 30 destinée à l'ancrage dans la vertèbre, surmontée par une tête 31 en forme de U destinée à recevoir l'élément de stabilisation dynamique 1, le fond du U définissant une zone de réception de l'élément de stabilisation dynamique 1. L'élément de stabilisation dynamique 1 est maintenu en place dans le fond du U de la tête 31 au moyen d'une pièce de fermeture 32. La tête 31 du moyen d'ancrage 3 et la pièce de fermeture 32 sont configurées pour coopérer mutuellement par encliquetage.

Le moyen de serrage 4 de l'élément de stabilisation dynamique 1 dans la tête 3 consiste en un élément formant écrou ou vis de serrage destiné à être logé dans une cavité traversante ménagée dans la pièce de fermeture 32. Lorsqu'il est logé dans la cavité de la pièce de fermeture 32, le moyen de serrage 4 vient en appui contre l'élément de stabilisation dynamique 1, serrant ledit élément contre le fond du U de la tête 31. Avantageusement, la cavité présente une forme complémentaire à celle du moyen de serrage 4.

Il est bien entendu évident que la configuration des moyens d'ancrage est donnée à titre d'exemple et que l'invention ne se limite pas à une telle configuration. En particulier, il peut être prévu que la tête 31 constitue une pièce distincte des moyens d'ancrage 3, du type un connecteur en soi conventionnel dans les systèmes de connexion rachidienne.

L'élément de stabilisation dynamique 1 se présente sous la forme d'une tige 5 s'étendant selon un axe longitudinal A, laquelle tige comprend un câble 6 entouré par une enveloppe 7 en matériau élastique. Cette constitution permet ainsi de conférer la flexibilité nécessaire pour permettre une liaison dynamique des vertèbres entre elles. Avantageusement, le câble est en Titane et l'enveloppe 7 en polymère, tel que du Polycarbonate Uréthane.

L'élément de liaison 1 comporte en outre une gaine de fixation 8 comprenant des zones rigides 9 disposées successivement les unes à la suite des autres. Ces zones rigides 9 sont espacées les unes des autres à une distance suffisante pour autoriser notamment un mouvement de flexion de ladite tige 5. Le comportement de flexibilité de la tige 5 est ainsi préservé.

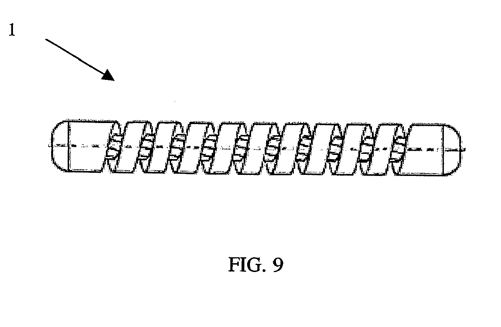

Dans le mode de réalisation décrit, la gaine de fixation 8 consiste en des anneaux 10 indépendants et distincts, fixes sur l'enveloppe 7. Il est bien entendu évident qu'il s'agit d'un exemple particulier de réalisation, la gaine de fixation 8 pouvant présenter tout autre agencement permettant la formation de zones rigides espacées, comme par exemple une gaine en forme hélicoïdale (figure 9).

Comme on l'a vu précédemment, la tige 5 est disposée dans le fond du U de la tête 31 des moyens d'ancrage 3 puis maintenue « fixée » dans celle-ci par le moyen de serrage 4, lequel vient en contact par appui sur la tige 5. Le moyen de serrage 4 définit, avec la tige 5, une zone de contact 11. La zone de contact 11 est caractérisée par sa longueur nominale.

Afin que la force de serrage des moyens de serrage 4 s'exerce essentiellement sur les anneaux 10, la distance séparant lesdits anneaux 10 est déterminée de façon à être inférieure à la longueur nominale de la zone de contact 11. Ainsi, la tige 5 ne nécessite pas un placement spécifique sur les ensembles de connexion, le moyen de serrage de chaque ensemble de connexion exerçant une pression principalement sur les anneaux 10 quelque soit leur position sur la tige 5.

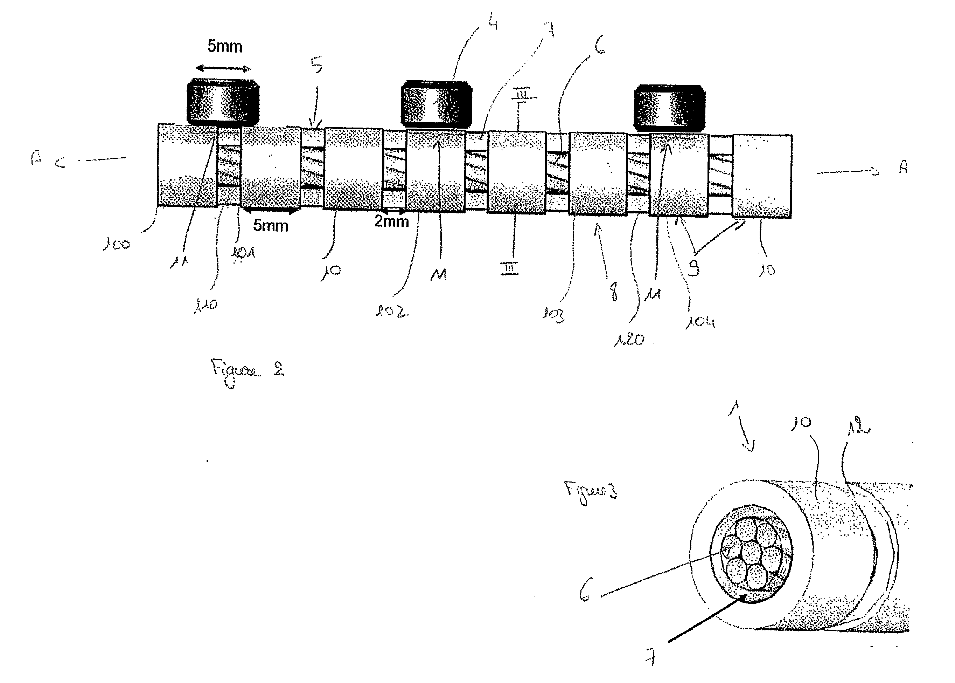

La figure 2 illustre un exemple de configuration d'une telle tige où, pour faciliter la compréhension, seuls les moyens de serrage 4 de trois ensembles de connexion ont été représentés. Dans cet exemple, la tige 5 comprend des anneaux 10 présentant une longueur de 5 millimètres. Ces anneaux 10 sont disposés sur l'enveloppe 7 de la tige 5, à distance régulière les uns des autres. Chaque anneau 10 est espacé avec les anneaux adjacents d'une distance de 2 millimètres. Les moyens de serrage 4 représentés sont de forme sensiblement circulaire. La face de contact des moyens de serrage 4 avec l'élément de correction 1 présente avantageusement un diamètre de 5 millimètres. Les moyens de serrage 4, en appui sur la tige 5, forment des zones de contact 11 présentant une longueur nominale de 5 millimètres, soit une longueur supérieure à l'écartement fixé entre chacun des anneaux 10. Il s'ensuit que, quelle que soit la position des moyens de serrage 4 sur la tige 5, la pression appliquée par les moyens de serrage 4 sur la tige 5 s'exerce sur les anneaux 10.

Les anneaux 10, et par extension la gaine de fixation 8, sont maintenus bloqués sur l'enveloppe 7. En effet, lors de la fabrication de l'élément de stabilisation dynamique 1, la matière qui forme par la suite l'enveloppe 7 s'écoule dans les espaces formés entre le câble et les anneaux 10. Les anneaux 10 sont alors maintenus écartés les uns des autres et bloqués par des renflements 12 radiaux de l'enveloppe 7 formées entre lesdits anneaux (figure 3).

La présence des renflements 12 de matière plastique offre un double avantage. D'une part, les renflements 12 permettent, comme on vient de le voir, d'emprisonner les anneaux, évitant leur glissement sur l'enveloppe 7. D'autre part, les renflements 12, disposées entre les zones rigides de la gaine de fixation, constituent respectivement des zones d'amortissement du mouvement en extension, compression et flexion de l'élément de stabilisation dynamique 1.

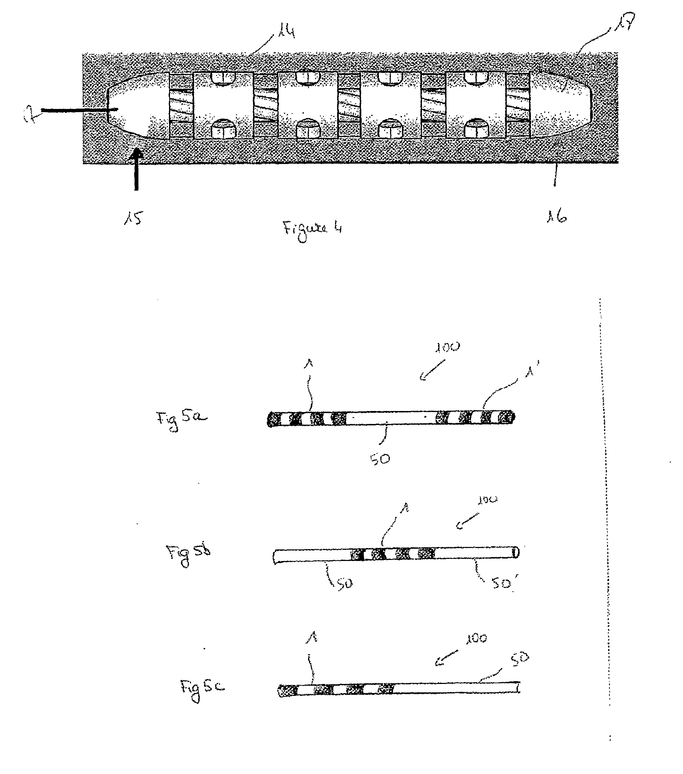

Selon une configuration avantageuse, les anneaux 10 comportent des lumières 14 (figure 4). La présence de ces lumières permet d'améliorer le maintien des anneaux 10 sur la tige 5. Elles viennent renforcer la fonction de maintien des renflements 12 de l'enveloppe 7.

La figure 2 illustre l'ensemble des possibilités de positionnement des moyens de serrage 4 sur la tige 5. Le premier moyen de serrage (situé le plus à gauche sur la tige) est disposé chevauchant deux anneaux 100, 101 adjacents de la tige 5. Ce premier moyen de serrage présente donc une surface de contact recouvrant des portions de deux anneaux voisins 100, 101 et l'espacement 110 ménagé entre les deux anneaux 100, 101. De part leur rigidité, la force de serrage s'exerce sur les anneaux 100, 101. Le deuxième moyen de serrage (moyen de serrage central) est entièrement en contact avec un anneau de la tige 5 (anneau 102). La force de serrage exercée par le moyen de serrage central s'applique donc sur l'anneau concerné seul. Le troisième moyen de serrage (situé le plus à droite) est disposé chevauchant partiellement l'espacement 120 ménagé entre les anneaux 103, 104 et ledit un anneau 104. Là encore, du fait de sa rigidité, la force de serrage ne s'exerce que sur l'anneau 104.

Les moyens de serrage 4 ainsi agencés assurent un serrage et un maintien suffisant de la tige 5 sur les moyens d'ancrage 3.

Par ailleurs, et avantageusement, les extrémités libres 15, 16 de l'élément de stabilisation dynamique 1 sont pourvues respectivement d'un embout rigide 17, 18. Selon un mode de réalisation particulier, les embouts 17, 18 sont fixés, de préférence par soudage ou emboutissage, auxdites extrémités.

Selon une configuration particulière, un ou plusieurs anneaux sont montés coulissants sur la tige 5.

Les figures 1 à 4 illustrent un élément de stabilisation dynamique comprenant une gaine de fixation 7 dont les zones rigides 9 présentent une même longueur et sont équidistantes les unes des autres. Il est bien entendu évident que l'invention ne se limite pas à une telle configuration et qu'il peut être prévu des zones rigides présentant des dimensions différentes et/ou les zones rigides présentant des espacements pouvant différer d'un anneau à un autre (non représenté), étant entendu que la distance entre chaque anneau devant rester inférieure à la longueur nominale de la zone de contact.

Par ailleurs, il peut être prévu que l'élément de stabilisation dynamique 1 soit prolongé à l'une au moins de ses extrémités par un élément rigide (figure 5b et figure 5c), lui-même pouvant être prolongé par un autre élément de stabilisation dynamique 1' (figure 5a), de sorte à former un élément de liaison 100 hybride assurant à la fois une liaison d'ostéosynthèse et une liaison dynamique. Plus particulièrement, l'élément de liaison 100 de la figure 5a est pourvu de deux parties 1, 1' assurant une liaison dynamique reliées par une partie assurant une liaison d'ostéosynthèse 50. L'élément de liaison 100 de la figure 5b illustre un élément de liaison pourvu de deux parties 50, 50' assurant une liaison d'ostéosynthèse reliées par une partie assurant une liaison dynamique 1. Il est bien entendu évident que l'invention ne se limite pas à un tel agencement d'éléments, et qu'il pourra être prévu un élément de liaison constitué par une succession d'éléments de liaison d'ostéosynthèse et d'éléments dynamiques.

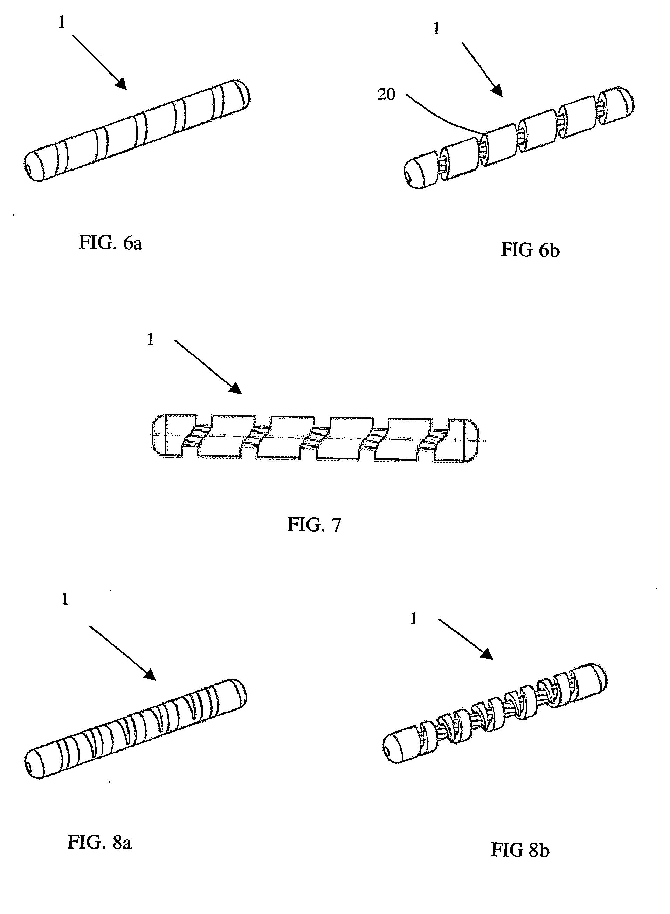

Dans le mode de réalisation précédemment décrit, la gaine de fixation 8 est formée par des anneaux 10, chacun des anneaux 10 présentant des faces d'extrémité 20 perpendiculaires à l'axe longitudinal A de la tige 5. Afin d'améliorer la résistance de l'élément de liaison dynamique 1 à la torsion, il est avantageux de prévoir des anneaux 10 configurés pour présenter respectivement des faces d'extrémités 20 inclinées par rapport à l'axe longitudinal A de la tige 5, les faces d'extrémité 20 de chaque anneau 10 étant disposées parallèles l'une par rapport à l'autre (figures 6a et 6b).

Sur la figure 6b, les faces d'extrémité 20 sont représentées planes. Il est bien entendu évident qu'il s'agit d'un exemple particulier de réalisation, les extrémités de chaque anneau 10 pouvant présenter une surface quelconque, comme illustré à titre d'exemple sur la figure 7.

Il peut être également prévu des anneaux 10 en forme hélicoïdale (figures 8a et 8b). Cette configuration de la gaine de fixation 8 permet d'améliorer les propriétés de compression et d'extension de l'élément de liaison dynamique 1. Avantageusement, le pas hélicoïdal et le nombre de spires des anneaux 10 ainsi configurés seront définis suivant le comportement souhaité de l'élément de liaison dynamique 1 en réponse à une sollicitation en compression ou en extension.

L'invention est décrite dans ce qui précède à titre d'exemple. Il est entendu que l'homme du métier est à même de réaliser différentes variantes de l'invention sans pour autant sortir de l'invention qui est définie par les revendications.

## Revendications

1. Elément de stabilisation dynamique (1) pour vertèbres apte à coopérer avec au moins deux ensembles de connexion implantables (2), chaque ensemble de connexion comprenant un moyen d'ancrage (3) dans une vertèbre agencé pour recevoir l'élément de stabilisation dynamique (1) et un moyen de serrage (4) de l'élément de stabilisation dynamique (1) sur ledit moyen d'ancrage (3), l'élément de stabilisation dynamique (1) comprenant une tige (5) s'étendant selon un axe longitudinal et comportant un câble (6) pourvu d'une enveloppe (7) en matériau élastique, l'élément de stabilisation dynamique comprenant une gaine de fixation (8) comportant des zones rigides (9) espacées les unes des autres et entourant la tige (5), et des anneaux d'amortissement d'un mouvement de l'élément (1), (7) disposés respectivement entre deux zones rigides (9) adjacentes de la gaine de fixation, **caractérisé en ce que** les anneaux d'amortissement sont constitués par des renflements (12) radiaux de l'enveloppe.

2. Elément de stabilisation dynamique (1) selon la revendication 1, **caractérisé en ce que** les zones rigides (9) sont espacées les unes des autres d'une distance inférieure à la longueur nominale d'une zone de contact (11) définie par le moyen de serrage (4) avec l'élément de stabilisation dynamique (1).

3. Elément de stabilisation dynamique (1) selon la revendication 1 ou la revendication 2, **caractérisé en ce que** les zones rigides (9) consistent en des anneaux (10) distincts.

4. Elément de stabilisation dynamique (1) selon la revendication 1 ou la revendication 2, **caractérisé en ce que** la gaine de fixation consiste en une bande hélicoïdale comprenant des spires s'étendant autour de la tige (5) selon un axe sensiblement coaxial avec l'axe longitudinal de ladite tige, lesdites spires formant les zones rigides de la gaine de fixation.

5. Elément de stabilisation dynamique (1) selon l'une quelconque des revendications précédentes, **caractérisé en ce que** les zones rigides (9) comprennent une ou plusieurs lumières (14) pour favoriser la fixation de la gaine de fixation sur l'enveloppe (7).

6. Elément de stabilisation dynamique (1) selon l'une quelconque des revendications précédentes, **caractérisé en ce que** les zones rigides (9) sont équidistantes les unes des autres.

7. Elément de stabilisation dynamique (1) selon l'une quelconque des revendications précédentes, **caractérisé en ce qu'**il comprend des moyens de maintien de la gaine de fixation sur la tige (5), les moyens de maintien de la gaine de fixation étant formés par les anneaux d'amortissement.

8. Elément de stabilisation dynamique (1) selon l'une quelconque des revendications précédentes, **caractérisé en ce qu'**il comprend des embouts (17,18) rigides, fixés à chaque extrémité (15, 16) du câble.

9. Elément de liaison comprenant au moins un élément de stabilisation dynamique (1) selon l'une quelconque des revendications précédentes, l'élément de stabilisation dynamique (1) étant prolongé par au moins une tige rigide.

10. Système de fixation rachidien comportant au moins deux ensembles de connexion rachidienne implantables (2), les deux ensembles au moins étant reliés par un élément de stabilisation dynamique (1) selon l'une quelconque des revendications 1 à 9.

## Patentansprüche

1. Dynamisches Stabilisierungselement (1) für Wirbel, das geeignet ist, mit wenigstens zwei implantierbaren Verbindungsstrukturen (2) zusammenzuwirken, wobei jede Verbindungsstruktur ein Verankerungsmittel (3) in einem Wirbel umfasst, das angeordnet ist, um das dynamische Stabilisierungselement (1) aufzunehmen, und ein Einspannmittel (4) des dynamischen Verbindungselements (1) auf dem genannten Verankerungsmittel (3), wobei das dynamische Verbindungselement (1) einen Stift (5) umfasst, der sich gemäß einer Längsachse erstreckt und ein Kabel (6) umfasst, das mit einem Umschlag (7) aus elastischem Material versehen ist, wobei das dynamische Verbindungselement eine Befestigungshülle (8) mit steifen Bereichen (9) umfasst, die voneinander beabstandet sind und den Stift (5) umgeben, und Dämpferringe einer Bewegung des Elements (1), die jeweils zwischen zwei, an der Befestigungshülle anliegenden steifen Bereichen (8) angeordnet sind, **dadurch gekennzeichnet, dass** die Dämpferringe durch radiale Verdickungen (12) des Umschlags (7) gebildet sind.

2. Dynamisches Stabilisierungselement (1) gemäß Anspruch 1, **dadurch gekennzeichnet, dass** die steifen Bereiche (9) voneinander um einen kleineren Abstand als die nominale Länge eines Kontaktbereichs (11) beabstandet sind, der durch das Einspannmittel (4) mit dem dynamischen Stabilisierungselement (1) definiert wird.

3. Dynamisches Stabilisierungselement (1) gemäß Anspruch 1 oder Anspruch 2, **dadurch gekennzeichnet, dass** die steifen Bereiche (9) aus unterschiedlichen Ringen (10) bestehen.

4. Dynamisches Stabilisierungselement (1) gemäß Anspruch 1 oder Anspruch 2, **dadurch gekennzeichnet, dass** die Befestigungshülle aus einem schneckenförmigen Band gebildet ist, das Windungen umfasst, die sich gemäß einer deutlich mit der Längsachse des genannten Stiftes koaxialen Achse um den Stift (5) erstrecken, wobei die genannten Windungen die steifen Bereiche der Befestigungshülle bilden.

5. Dynamisches Stabilisierungselement (1) gemäß einem der voranstehenden Ansprüche, **dadurch gekennzeichnet, dass** die steifen Bereiche (9) eine oder mehrere Öffnungen (14) umfassen, um die Befestigung der Befestigungshülle auf dem Umschlag (7) zu erlauben.

6. Dynamisches Stabilisierungselement (1) gemäß einem der voranstehenden Ansprüche, **dadurch gekennzeichnet, dass** die steifen Bereiche (9) in gleichen Abständen voneinander beabstandet sind.

7. Dynamisches Stabilisierungselement (1) gemäß einem der voranstehenden Ansprüche, **dadurch gekennzeichnet, dass** es Festhaltemittel der Befestigungshülle auf dem Stift (5) umfasst, wobei die Festhaltemittel der Befestigungshülle durch Dämpferringe gebildet sind.

8. Dynamisches Stabilisierungselement (1) gemäß einem der voranstehenden Ansprüche, **dadurch gekennzeichnet, dass** es steife, an jedem Ende (15, 16) des Kabels befestigte Ansatzstücke (17, 18) umfasst.

9. Verbindungselement mit wenigstens einem dynamischen Stabilisierungselement (1) gemäß einem der voranstehenden Ansprüche, wobei das dynamische Stabilisierungselement (1) durch wenigstens einen steifen Stift verlängert wird.

10. Befestigungssystem der Wirbelsäule, das wenigstens zwei Gruppen von implantierbaren Verbindungen der Wirbelsäule (2) umfassen, wobei die zwei Strukturen wenigstens durch ein dynamisches Stabilisierungselement (1) gemäß Anspruch 1 bis 9 verbunden sind.

## Claims

1. A dynamic stabilization element (1) for vertebrae able to cooperate with at least two connection implantable assemblies (2), with each connection assembly comprising a means (3) for anchoring into a vertebra adapted for receiving the dynamic stabilisation member (1), and a means (4) for clamping the dynamic stabilisation element (1) on said anchoring means (3), with the dynamic stabilization element (1) comprising a rod (5) extending along a longitudinal axis and including a cable (6) provided with a casing (7) made of an elastic material, with the dynamic stabilization element comprising a fastening sheath (8) including rigid areas (9) spaced from each other and surrounding the rod (5), and rings for damping a movement of the element (1) positioned respectively between two adjacent rigid areas (9) of the fastening sheath, **characterised in that** the damping rings are constituted by radial bulges (12) of the casing.

2. A dynamic stabilization element (1) according to claim 1, **characterized in that** the rigid areas (9) are spaced from one another by a distance smaller than the nominal length of a contact area (11) defined by the clamping means (4) with the dynamic stabilization element (1).

3. A dynamic stabilization element (1) according to claim 1 or claim 2, **characterized in that** the rigid areas (9) consist of separate rings (10).

4. A dynamic stabilization element (1) according to claim 1 or claim 2, **characterized in that** the fastening sheath consists of a helical strip comprising turns extending around the rod (5) along an axis substantially coaxial with the longitudinal axis of said rod, with said turns forming the rigid areas of the fastening sheath.

5. A dynamic stabilization element (1) according to any one of the preceding claims, **characterized in that** the rigid zones (9) comprise one or more ports (14) to facilitate the attachment of the fastening sheath on the casing (7).

6. A dynamic stabilization element (1) according to any one of the preceding claims, **characterized in that** the rigid zones (9) are equidistant from each other.

7. A dynamic stabilization element (1) according to any one of the preceding claims, **characterized in that** it comprises means for holding the fastening sheath on the rod (5), with the means for holding the fastening sheath being formed by the damping rings.

8. A dynamic stabilization element (1) according to any one of the preceding claims, **characterized in that** it comprises rigid end-pieces (17, 18) attached to each end (15, 16) of the cable.

9. A connecting element comprising at least one dynamic stabilization element (1) according to any one of the preceding claims, with the dynamic stabilization element (1) being extended by at least one rigid rod.

10. A spinal fastening system comprising at least two spinal connection implantable assemblies (2), with the at least two assemblies being connected by a dynamic stabilization element (1) according to any one of claims 1 to 9.
